# EUROPÄISCHE PATENTANMELDUNG

(11) **EP 0 950 389 A2**
(43) Veröffentlichungstag der Anmeldung: **20.10.1999**
(21) Anmeldenummer: 99106982.4
(22) Anmeldetag: 09.04.1999
(51) Int. Cl.: A61F 2/44

(54) **Zwischenwirbelfusionsimplantat**

(30) Priorität: 16.04.1998 DE 19816828
(71) Anmelder: Aesculap AG & Co. KG, 78532 Tuttlingen (DE)
(72) Erfinder: Schlegel, Jörg, 72458 Albstadt (DE); Wing, Charles, 78573 Wurmlingen (DE)
(74) Vertreter: Böhme, Ulrich, Dr. Dipl.-Phys.

(57) **Zusammenfassung**

Um bei der Fusion von benachbarten Wirbelkörpern das Knochenwachstum im Zwischenwirbelraum zu stimulieren, wird ein Zwischenwirbelfusionsimplantat mit zwei Stützkörpern aus einem Material, dessen Steifigkeit größer ist als die des Knochenmaterials von Wirbelkörpern, vorgeschlagen, von denen jeder eine Stützfläche zur Anlage an einem von zwei benachbarten Wirbelkörpern aufweist, deren Fläche kleiner ist als die Querschnittsfläche der Wirbelkörper, welches gekennzeichnet ist durch eine Führung, durch die die beiden Stützkörper quer zu den Stützflächen unverkippbar gegeneinander verschiebbar geführt sind, und durch ein Federelement, das die beiden Stützkörper längs der Führung auseinanderschiebt.

## Beschreibung

Die Erfindung betrifft ein Zwischenwirbelfusionsimplantat mit zwei Stützkörpern aus einem Material, dessen Steifigkeit größer ist als die des Knochenmaterials von Wirbelkörpern, von denen jeder eine Stützfläche zur Anlage an einem von zwei benachbarten Wirbelkörpern aufweist, deren Fläche kleiner ist als die Querschnittsfläche der Wirbelkörper.

Bei Schäden an der Bandscheibe ist es bekannt, nach Ausschöpfung aller konservativer Therapiemöglichkeiten die Bandscheibe zwischen benachbarten Wirbelkörpern zu entfernen und durch ein Implantat zu ersetzen, das im wesentlichen die Funktion der Bandscheibe übernimmt, das also benachbarte Wirbelkörper gegeneinander abstützt und nach Möglichkeit deren relative Beweglichkeit erhält. Dazu werden Implantate zwischen die benachbarten Wirbelkörper eingesetzt, die zwei voneinander getrennte Stützkörper aufweisen, die sich mit einer Stützfläche an die benachbarten Wirbelkörper anlegen und zwischen denen elastisch komprimierbare Federelemente angeordnet sind, wobei die Lagerung so gewählt ist, daß die Stützflächen nicht nur elastisch einander annäherbar sind, sondern auch zu Erhaltung der Beweglichkeit der Wirbelkörper gegeneinander neigbar (EP 0 560 140 A1; US 5 458 642 A). Bei diesen Implantaten handelt es sich um Bandscheibenersatzimplantate.

Es sind weiterhin Implantate bekannt, die als Fusionsimplantate dienen, die also die benachbarten Wirbelkörper so relativ zueinander fixieren, daß die beiden Wirbelkörper im Laufe der Zeit durch eine Brücke aus Knochensubstanz dauerhaft miteinander verbunden werden (WO89/12431; US 5 609 636 A). Diese Fusionsimplantate sind sehr kräftig und damit auch steif ausgebildet, da sie die Wirbelkörper in definiertem Abstand zueinander fixieren sollen. Aufgrund dieser Konstruktionsweise verläuft die Hauptkraftkomponente durch das Implantat selbst, so daß sich dadurch eine Entlastung des umgebenden organischen Materials ergibt.

In der Praxis hat sich herausgestellt, daß das Knochenwachstum zwischen benachbarten Wirbelkörpern beim Einsetzen derartiger Fusionsimplantate nicht in allen Fällen in der gewünschten Weise schnell und dauerhaft erfolgt. Häufig bildet sich fibröses Gewebe statt Knochen in und um diese Implantate; dieses fibröse Gewebe hat nicht die Festigkeitseigenschaften von Knochen, und dies führt dazu, daß die zwischen benachbarten Wirbelkörpern wirkenden Kräfte nur über die sehr kleine Fläche der Implantatendflächen übertragen werden. Dies kann zu Endplatteneinbrüchen führen.

Es ist auch ein Zwischenwirbelimplantat bekannt, welches einstückig ausgebildet ist und welches in einem Umfangsteil Durchbrüche aufweist, um dadurch die Festigkeit herabzusetzen und eine Nachgiebigkeit zu erzeugen, die der der natürlichen Knochensubstanz entspricht (DE 195 41 114 A1). Bei einer solchen einteiligen Ausgestaltung ist es jedoch mechanisch nicht in kontrollierter Weise erreichbar, daß die gewünschte Nachgiebigkeit allein durch Durchbrechungen im Außenmantel des Implantats entsteht, außerdem besteht bei einem solchen Implantat die Gefahr, daß die Belastung am Umfang des Implantats ungleichmäßig angreift und dadurch zu einer Verkippung der Stützflächen führt.

Es ist Aufgabe der Erfindung, ein Wirbelkörperfusionsimplantat der eingangs beschriebenen Art so auszugestalten, daß das Knochenwachstum im Zwischenwirbelbereich gefördert wird und daß Endplatteneinbrüche vermieden werden.

Diese Aufgabe wird erfindungsgemäß durch ein Zwischenwirbelfusionsimplantat der eingangs beschriebenen Art gelöst, das gekennzeichnet ist durch eine Führung, durch die die beiden Stützkörper quer zu den Stützflächen unverkippbar gegeneinander verschiebbar geführt sind, und durch ein Federelement, das die beiden Stützkörper längs der Führung auseinanderschiebt.

Ein solches Zwischenwirbelfusionsimplantat unterscheidet sich grundlegend von Zwischenwirbelersatzimplantaten, die keine knöcherne Verbindung zwischen benachbarten Wirbelkörpern hervorrufen sollen und die daher normalerweise die Wirbelkörper an den einander zugewandten Seiten vollständig abdecken.

Bei dem Zwischenwirbelfusionsimplantat der vorliegenden Erfindung wird die Stützfläche der Stützkörper bewußt kleiner ausgeführt als die Querschnittsfläche der Wirbelkörper, so daß seitlich von den Stützflächen knöcherne Brücken zum benachbarten Wirbelkörper ausgebildet werden können, die Zwischenwirbelfusionsimplantate sollen nach Möglichkeit allseits von Knochenmaterial umwachsen werden.

Ganz wesentlich ist die Führung der beiden Stützkörper gegeneinander, dadurch sind die Stützkörper nur in der direkten Verbindungsrichtung der beiden Wirbelkörper gegeneinander verschiebbar, die Neigung der Stützflächen gegeneinander bleibt aber bei dieser Verschiebung unverändert.

Die Stützkörper werden dabei so kräftig durch das Federelement auseinandergeschoben, daß auch im Bereich der knöchernen Verbindung zwischen den Wirbelkörpern eine geringfügige Zusammendrückbarkeit erhalten wird, und diese Zusammendrückbarkeit führt dazu, daß die knöcherne Substanz zwischen benachbarten Wirbelkörpern mit wechselndem Druck beaufschlagt und dadurch zu weiterem Knochenwachstum stimuliert wird. Es werden also nicht die gesamten Druckkräfte zwischen den Wirbelkörpern allein durch das Zwischenwirbelfusionsimplantat aufgenommen, sondern ein Teil der anstehenden Kräfte werden über die Knochenbrücke geleitet, die dadurch zum Knochenwachstum angeregt wird.

Wenn hier von der Ausfüllung einer Knochenbrücke zwischen benachbarten Wirbelkörpern gesprochen wird, kann es sich dabei auch um Wirbelkörper handeln, die normalerweise nicht unmittelbar benachbart sind, sondern die nur dadurch benachbart werden, daß zwischen ihnen ein Wirbelkörper entfernt wird. In diesem Falle wird das Wirbelfusionsimplantat entsprechend größer dimensioniert, so daß es den fehlenden Wirbelkörper ersetzt und eine knöcherne Brücke zwischen den nunmehr benachbarten Wirbelkörpern ausbildet.

Vorzugsweise ist das Federelement derart ausgelegt, daß sich die Implantathöhe nach dem Einsetzen des oder der Implantate zwischen die Wirbelkörper bei Einwirkung einer in Verbindungsrichtung der beiden Wirbelkörper wirkenden Druckkraft von 3 KN um 3 % bis 20 % ändert. Dieser Änderung wirken einmal entgegen das oder die eingesetzten Implantate und zum anderen auch die sich zwischen den Wirbelkörpern ausbildende Knochenbrücke.

Das Federelement kann beispielsweise eine Steifigkeit aufweisen, die zwischen 1 GN/m² und 10 GN/m² liegt, insbesondere zwischen 1 und 3 GN/m².

Das Federelement kann sehr unterschiedlich ausgebildet sein, beispielsweise kann gemäß einer bevorzugten Ausführungsform vorgesehen sein, daß das Federelement ein zwischen die beiden Stützkörper eingesetztes Knochenstück ist. Dieses Knochenstück wird beidseitig von den beiden Stützkörpern beaufschlagt und hält dadurch diese Stützkörper in gegenseitigem Abstand, wobei die Zusammendrückbarkeit des Implantates durch die elastischen Eigenschaften des Knochenmaterials selbst bestimmt werden.

Bei einer anderen bevorzugten Ausführungsform kann das Federelement eine Schraubenfeder, eine Tellerfeder oder eine Biegefeder sein, grundsätzlich ist auch das Einsetzen eines elastisch zusammendrückbaren Polsters oder Stützkörpers möglich, wobei dann entsprechendes Material für dieses Stützpolster verwendet wird, welches in seiner Zusammendrückbarkeit und Steifigkeit im wesentlichen dem Knochenmaterial entspricht.

Bei einer ersten bevorzugten Ausführungsform eines Zwischenwirbelfusionsimplantates ist vorgesehen, daß die beiden Stützkörper mit rohrförmigen Ansätzen teleskopierend ineinander geführt sind.

Es ist dann vorteilhaft, wenn im Innenraum der rohrförmigen Ansätze eine Schraubenfeder angeordnet ist, die die beiden Stützkörper auseinanderschiebt.

Bei einer anderen Ausführungsform kann vorgesehen sein, daß ein Stützkörper topfförmig ausgebildet ist, daß in diesen eine Führungshülse des anderen Stützkörpers hineinragt und daß eine Druckfeder im Ringraum zwischen der Führungshülse und der Außenwand des topfförmigen Stützkörpers angeordnet ist.

Dabei ist es günstig, wenn die Druckfeder die beiden Stützkörper längs ihres Verschiebeweges führt, es können aber auch andere Führungsmittel vorgesehen sein, die die beiden gegeneinander verschiebbaren Teile uniaxial relativ zueinander führen.

Es ist dabei vorteilhaft, wenn die Stützfläche der beiden Stützkörper ringförmig ausgebildet ist, auf diese Weise steht auch der Innenraum der Implantates für das Knochenwachstum zwischen den Wirbelkörpern zur Verfügung.

Bei den bisher beschriebenen Ausführungsformen ist das Implantat mindestens dreiteilig ausgebildet, das Federelement bildet nämlich neben den beiden Stützkörpern ein separates Teil.

Bei einer abgewandelten Ausführungsform ist es auch möglich, daß das Federelement Teil mindestens einer der beiden Stützkörper ist. Es können also entweder ein Stützkörper oder beide Stützkörper so ausgebildet sein, daß sie selbst elastisch zusammendrückbar sind und somit die Funktion des Federelementes zusätzlich übernehmen.

Beispielsweise kann vorgesehen sein, daß mindestens ei-ner der beiden Stützkörper einen federnden Abschnitt aufweist, der durch in axialem Abstand angeordnete horizontale Einschnitte in seinem Querschnitt geschwächt ist.

Dabei ist es vorteilhaft, wenn die Einschnitte in einer Ebene von gegenüberliegenden Seiten her erfolgen und zwischen sich einen schmalen Materialsteg stehenlassen, und wenn die Einschnitte in benachbarten Ebenen winkelmäßig gegeneinander versetzt sind, beispielsweise um jeweils 90°. Es ergibt sich bei einer solchen Ausgestaltung eine elastische Zusammendrückbarkeit eines derartigen Abschnittes, die wesentlich größer ist als die durch die Materialeigenschaften des Stützkörpers vorgegebene Zusammendrückbarkeit, diese Zusammendrückbarkeit kann in der Größenordnung der Zusammendrückbarkeit des Knochenmaterials liegen.

Die Stützflächen sind vorzugsweise mit einer knochenfreundlichen Oberfläche versehen, d. h. mit einer Oberfläche, die das Knochenwachstum fördert und das Anwachsen von Knochenmaterial begünstigt.

Beispielsweise können die Stützflächen mit Hydroxylapatit beschichtet sein.

Vorteilhaft ist es auch, wenn die Seitenflächen der Stützkörper mit einer solchen knochenfreundlichen Oberfläche versehen sind.

Die Stützfläche kann auch eine aufgerauhte Oberfläche aufweisen.

Um ein möglichst vollständiges Knochenwachstum zwischen benachbarten Wirbelkörpern zu ermöglichen, ist es wichtig, daß genügend Fläche für dieses Knochenwachstum zur Verfügung steht. Es kann daher auch vorgesehen sein, daß in einem Zwischenwirbelraum zwei derartige Implantate nebeneinander eingesetzt sind, so daß auch im Zwischenraum zwischen den Implantaten Knochenwachstum auftreten kann.

Die nachfolgende Beschreibung bevorzugter Ausführungsformen der Erfindung dient im Zusammenhang mit der Zeichnung der näheren Erläuterung. Es zeigen:
- Figur 1:: eine schematische Vorderansicht von zwei benachbarten Wirbelkörpern mit einem Zwischenwirbelfusionsimplantat und mit dieses Implantat umgebendem knöchernem Material;
- Figur 2:: eine Ansicht ähnlich Fig. 1 mit zwei im Abstand zueinander angeordneten Zwischenwirbelfusionsimplantaten;
- Figur 3:: eine Ansicht ähnlich Fig. 1 mit einem verschieden aufgebauten Zwischenwirbel-fusionsimplantat;
- Figur 4:: eine Querschnittansicht eines anderen bevorzugten Fusionsimplantates im eingesetzten Zustand und
- Figur 5:: eine Schnittansicht längs Linie 5-5 in Figur 4.

Der Zwischenwirbelraum 1 zwischen zwei benachbarten Wirbelkörpern 2, 3 wird an seiner Oberseite und an seiner Unterseite jeweils begrenzt durch eine im wesentlichen ebene Endfläche 4, 5 der beiden Wirbelkörper 2 bzw. 3.

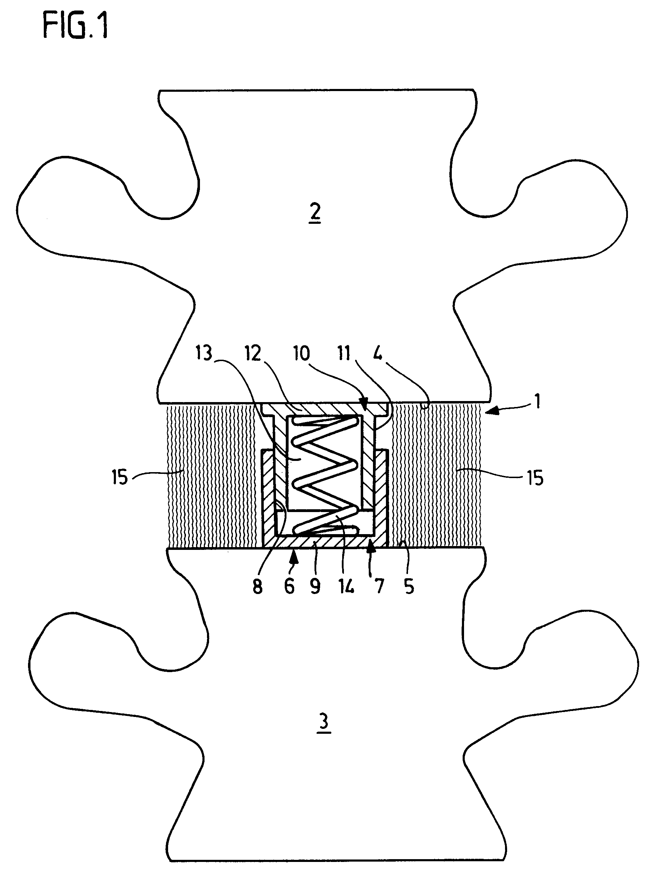

In diesen Zwischenwirbelraum 1 ist bei dem Ausführungsbeispiel der Fig. 1 ein Fusionsimplantat 6 eingesetzt, welches einen unteren Stützkörper 7 mit einer zylindrischen Außenwand und einem ebenen Boden 9 sowie einen oberen Stützkörper 10 mit einer zylindrischen Führungshülse 11 und einem ebenen Deckel 12 umfaßt. Der Boden 9 liegt an der Endfläche 5 des unteren Wirbelkörpers 3 an und bildet dort eine Stützfläche, in gleicher Weise liegt der Deckel 12 an der Endfläche 4 des oberen Wirbelkörpers 2 an und bildet dort eine Stützfläche. Die Führungshülse 11 greift derart in die zylindrische Aussenwand 8 ein, daß die beiden Teile flächig aneinander anliegen und eine Teleskopführung ausbilden, so daß die beiden Stützkörper 7 und 10 uniaxial gegeneinander verschiebbar geführt sind.

In dem von den beiden Stützkörpern 7 und 10 umgebenen Innenraum 13 ist eine Schraubenfeder 14 angeordnet, die sich einerseits am Boden 9 und andererseits am Deckel 12 abstützt. Sie drückt die beiden Stützkörper 7 und 10 auseinander und damit gegen die Endflächen 5 bzw. 4 der beiden Wirbelkörper, die dadurch ebenfalls auseinandergedrückt werden.

Die Fläche der Stützflächen, die durch den Boden 9 einerseits und den Deckel 12 andererseits gebildet werden, ist kleiner als die Endflächen 4, 5, im Ausführungsbeispiel der Fig. 1 ist das Fusionsimplantat 6 zentral in den Zwischenwirbelraum 1 eingeschoben, so daß zu den Rändern der Wirbelkörper hin im Zwischenwirbelraum 1 Platz für das Einfüllen von Knochenmaterial bleibt, das dort das Fusionsimplantat 6 umgebend eine knöcherne Brücke 15 zwischen den beiden benachbarten Wirbelkörpern 2, 3 ausbildet.

Die Steifigkeit der Schraubenfeder 14 ist an die Steifigkeit des im Bereich dieser Brücke 15 sich bildenden Knochenmaterials angepaßt, so daß vor der Ausbildung der knöchernen Brücke 15 zwar das Fusionsimplantat 6 die beiden Wirbelkörper in ausreichender Weise voneinander im Abstand hält, nach der Ausbildung der Brücke 15 jedoch das Fusionsimplantat 6 doch so weit kompressibel ist, daß beim Zusammendrücken der Wirbelkörper auch das Material der knöchernen Brücke 15 mit Druckkräften beaufschlagt wird. Diese periodische und wechselnde Beaufschlagung des Knochenmaterials führt zu einer Stimulation des Knochenwachstums, andererseits ist aber das Fusionsimplantat 6 kräftig genug, das Material der Brücke 15 gegen zu große Druckbeanspruchungen zuverlässig zu schützen.

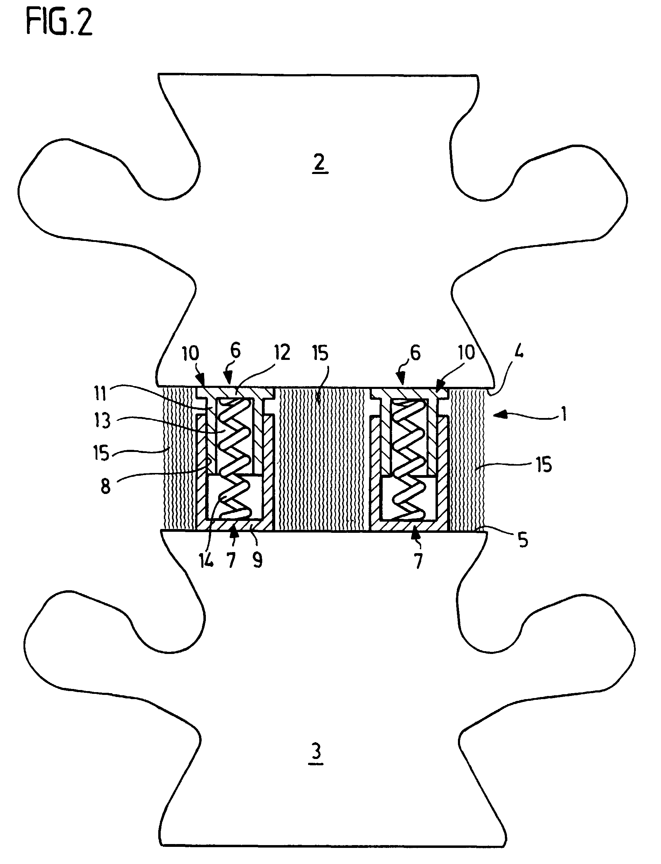

Bei dem Ausführungsbeispiel der Fig. 2 sind zwei gleich aufgebaute Fusionsimplantate 6 verwendet, deren Querabmessungen sind jedoch geringer als im Fall des Ausführungsbeispiels der Fig. 1, so daß im Zwischenwirbelraum 1 Platz bleibt, zwei derartige Fusionsimplantate 6 im Abstand zueinander im Zwischenwirbelraum 1 anzuordnen. Dadurch kann auch im zentralen Bereich eine knöcherne Brücke 15 ausgebildet werden.

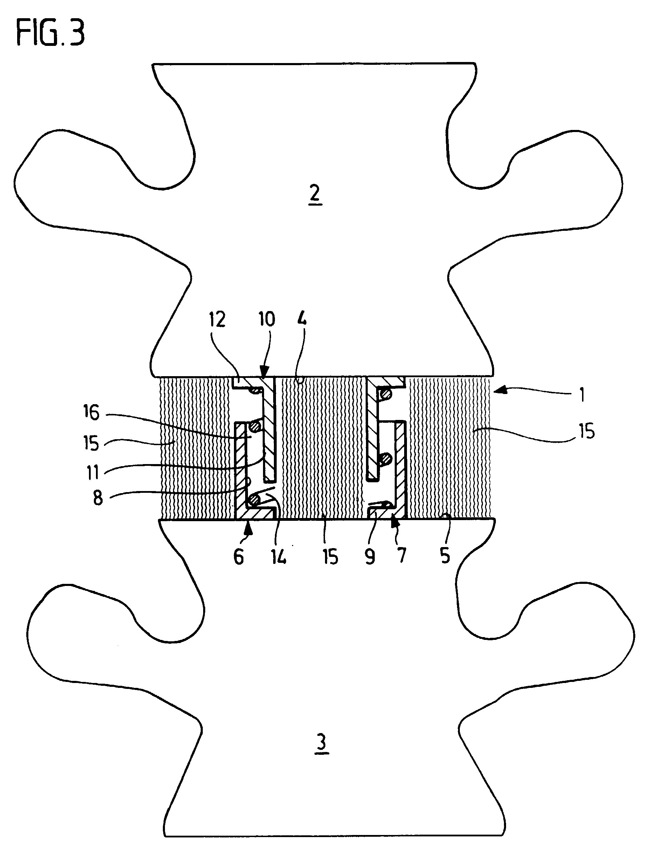

Bei dem Ausführungsbeispiel der Fig. 3 ist eine zentrale Anordnung des Fusionsimplantates 6 gewählt wie beim Ausführungsbeispiel der Fig. 1.

Im Unterschied zu dem Ausführungsbeispiel der Fig. 1 liegen hier die Führungshülse 11 und die zylindrische Außenwand 8 nicht flächig aneinander, sondern sie sind im Abstand zueinander angeordnet und bilden dadurch zwischen sich einen Ringraum 16 aus, in den die Schraubenfeder 14 eingelegt ist. Diese Schraubenfeder liegt sowohl an der Außenwand 8 als auch an der Führungshülse 11 an und führt somit die beiden Stützkörper 7 und 10 relativ zueinander. Durch die Anordnung der Schraubenfeder 14 im Ringraum 16 kann der zentrale Bereich des Fusionsimplantates 6 für die knöcherne Durchwachsung genutzt werden, aus diesem Grunde sind bei diesem Fusionsimplantat 6 im Unterschied zu dem der Figuren 1 und 2 sowohl der Boden 9 als auch der Deckel 12 ringförmig ausgebildet. Der dadurch entstehende Innenraum kann beim Einsetzen des Fusionsimplantates 6 mit knöchernem Material ausgefüllt werden, so daß die knöcherne Durchwachsung des Zwischenwirbelraums 1 gefördert wird.

Es wäre auch möglich, statt der Schraubenfeder 14 bei einem Implantat, wie es in Figur 1 dargestellt ist, ein anderes Federelement einzusetzen, beispielsweise eine Tellerfeder oder ein Tellerfedernpaket oder aber auch ein Knochenstück, beispielsweise in Form eines Kreiszylinders.

Die Implantate der Figuren 1 bis 3 sind dreiteilige Implantate mit zwei gegeneinander verschieblichen und gegeneinander geführten Stützkörpern und einem zwischen den beiden wirksamen, als getrenntes Teil ausgebildeten Federelement.

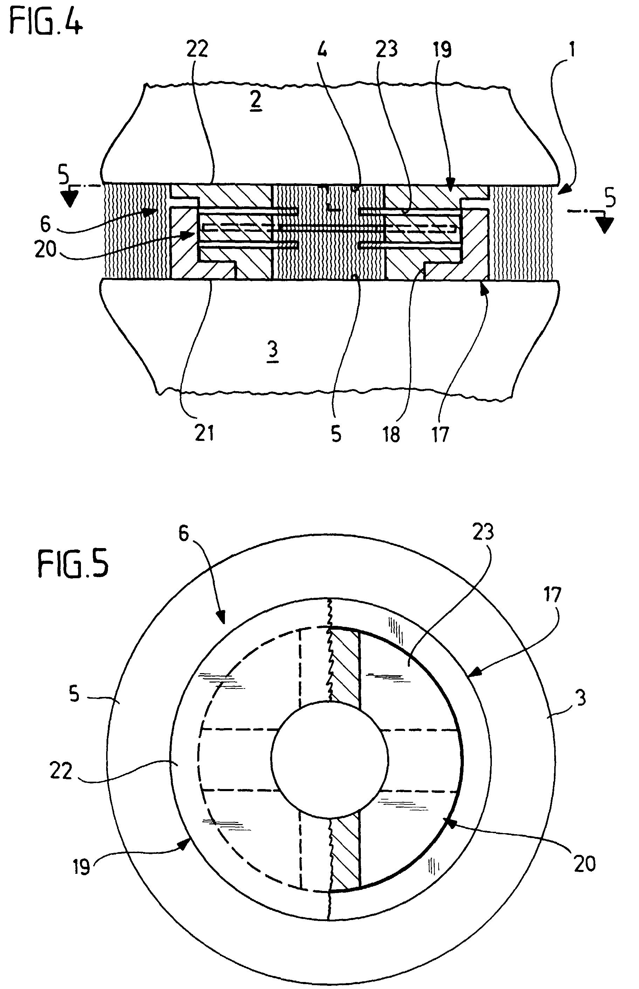

Bei dem Ausführungsbeispiel der Figuren 4 und 5 hingegen besteht das Implantat nur aus zwei Teilen, nämlich einem topfförmigen Stützkörper 17 mit einer großen zentralen Öffnung 18 und einem in diesen Stützkörper 17 eintauchenden zweiten Stützkörper 19, der mit seinem in den Stützkörper 17 und die Öffnung 18 eintauchenden Abschnitt 20 im Stützkörper 17 in axialer Richtung geführt ist. Die Unterseite 21 des Stützkörpers 17 bildet eine erste Stützfläche aus, die Oberseite 22 des Stützkörpers 19 eine zweite Stützfläche, diese Stützflächen werden an die Endflächen 5 bzw. 4 der Wirbelkörper 3 bzw. 2 angelegt.

In den Abschnitt 20 des Stützkörpers 19 sind quer zur Längsrichtung des zylindrischen Abschnittes 20 von außen her parallel zueinander verlaufende Einschnitte 23 eingearbeitet, die in einer Ebene jeweils von außen nach innen so weit eintreten, daß zwischen ihnen nur ein relativ schmaler Materialsteg an dem ringförmigen Abschnitt 20 verbleibt.

In benachbarten Ebenen sind die Einschnitte 23 in Umfangsrichtung um 90° gegeneinander versetzt, so daß insgesamt der Abschnitt 20 durch diese Einschnitte 23 so geschwächt wird, daß der Stützkörper 19 im Bereich des Abschnittes 20 trotz der hohen Steifigkeit des verwendeten Stützkörpermaterials in der Verbindungsrichtung der benachbarten Wirbelkörper elastisch zusammendrückbar wird. Die Dimensionierung ist dabei so gewählt, daß die Zusammendrückbarkeit in der Größenordnung der Zusammendrückbarkeit des Knochenmaterials liegt.

Damit übernimmt der Abschnitt 20 des Stützkörpers 19 die Aufgabe des Federelementes, das bei den Ausführungsbeispielen der Figuren 1 bis 3 durch ein separates Teil gebildet wird.

Auch bei diesem Implantat kann das Knochenmaterial an der Außenseite des Implantates vorbei und durch den offenen Innenraum hindurchwachsen, so daß die gewünschte knöcherne Überbrückung der benachbarten Wirbelkörper erreicht wird.

## Patentansprüche

1. Zwischenwirbelfusionsimplantat mit zwei Stützkörpern aus einem Material, dessen Steifigkeit größer ist als die des Knochenmaterials von Wirbelkörpern, von denen jeder eine Stützfläche zur Anlage an einem von zwei benachbarten Wirbelkörpern aufweist, deren Fläche kleiner ist als die Querschnittsfläche der Wirbelkörper, gekennzeichnet durch eine Führung (8, 11; 8, 14, 11; 17, 20), durch die die beiden Stützkörper (7, 10; 17, 19) quer zu den Stützflächen (9, 12; 21, 22) unverkippbar gegeneinander verschiebbar geführt sind, und durch ein Federelement (14; 20), das die beiden Stützkörper (7, 10; 17, 19) längs der Führung auseinanderschiebt.

2. Implantat nach Anspruch 1, dadurch gekennzeichnet, daß das Federelement (14; 20) derart ausgelegt ist, daß sich die Implantathöhe nach dem Einsetzen zwischen die Wirbelkörper (2, 3) bei Einwirkung einer in Verbindungsrichtung der beiden Wirbelkörper (2, 3) wirkenden Druckkraft von 3 KN um 3 % bis 20 % ändert.

3. Implantat nach Anspruch 1 oder 2, dadurch gekennzeichnet, daß das Federelement (14; 20) eine Steifigkeit aufweist, die zwischen 1 GN/m² und 10 GN/m² liegt.

4. Implantat nach Anspruch 3, dadurch gekennzeichnet, daß die Steifigkeit des Federelementes (14; 20) zwischen 1 und 3 GN/m² liegt.

5. Implantat nach einem der voranstehenden Ansprüche, dadurch gekennzeichnet, daß das Federelement (14; 20) ein zwischen die beiden Stützkörper (7, 10) eingesetztes Knochenstück ist.

6. Implantat nach einem der Ansprüche 1 bis 4, dadurch gekennzeichnet, daß das Federelement eine zwischen die beiden Stützkörper (7, 10) eingesetzte Schraubenfeder (14) ist.

7. Implantat nach einem der Ansprüche 1 bis 4, dadurch gekennzeichnet, daß das Federelement eine zwischen die beiden Stützkörper eingesetzte Tellerfeder ist.

8. Implantat nach einem der Ansprüche 1 bis 4, dadurch gekennzeichnet, daß das Federelement eine zwischen die Stützkörper eingesetzte Biegefeder ist.

9. Implantat nach einem der voranstehenden Ansprüche, dadurch gekennzeichnet, daß die beiden Stützkörper (7, 10) mit rohrförmigen Ansätzen (8, 11) teleskopierend ineinander geführt sind.

10. Implantat nach Anspruch 9, dadurch gekennzeichnet, daß im Innenraum (13) der rohrförmigen Ansätze (8, 11) eine Schraubenfeder (14) angeordnet ist, die die beiden Stützkörper (7, 10) auseinanderschiebt.

11. Implantat nach einem der Ansprüche 1 bis 8, dadurch gekennzeichnet, daß ein Stützkörper (7) topfförmig ausgebildet ist, daß in diesen eine Führungshülse (11) des anderen Stützkörpers (10) hineinragt und daß eine Druckfeder (14) im Ringraum (16) zwischen der Führungshülse (11) und der Außenwand (8) des topfförmigen Stützkörpers (7) angeordnet ist.

12. Implantat nach Anspruch 11, dadurch gekennzeichnet, daß die Druckfeder (14) die beiden Stützkörper (7, 10) längs ihres Verschiebeweges führt.

13. Implantat nach Anspruch 11 oder 12, dadurch gekennzeichnet, daß die Stützfläche (9, 12) der beiden Stützkörper (7, 10) ringförmig ausgebildet ist.

14. Implantat nach einem der Ansprüche 1 bis 4, dadurch gekennzeichnet, daß das Federelement Teil (20) mindestens eines der beiden Stützkörper (19) ist.

15. Implantat nach Anspruch 14, dadurch gekennzeichnet, daß mindestens einer der beiden Stützkörper (19) einen federnden Abschnitt (20) aufweist, der durch im axialen Abstand angeordnete horizontale Einschnitte (23) in seinem Querschnitt geschwächt ist.

16. Implantat nach Anspruch 15, dadurch gekennzeichnet, daß die Einschnitte (23) in einer Ebene von gegenüberliegenden Seiten her erfolgen und zwischen sich einen schmalen Materialsteg stehenlassen, und daß die Einschnitte (23) in benachbarten Ebenen winkelmäßig gegeneinander versetzt sind.

17. Implantat nach einem der voranstehenden Ansprüche, dadurch gekennzeichnet, daß die Stützflächen (9, 12; 21, 22) mit einer knochenfreundlichen Oberfläche versehen sind.

18. Implantat nach Anspruch 17, dadurch gekennzeichnet, daß die Stützflächen mit Hydroxylapatit beschichtet sind.

19. Implantat nach einem der voranstehenden Ansprüche, dadurch gekennzeichnet, daß die Seitenflächen der Stützkörper (7, 10; 17, 19) mit einer knochenfreundlichen Oberfläche versehen sind.

20. Implantat nach einem der voranstehenden Ansprüche, dadurch gekennzeichnet, daß die Stützflächen eine aufgerauhte Oberfläche aufweisen.
